(19) Europäisches Patentamt
European Patent Office
Office européen des brevets

(11) Publication number: **0 255 228 B1**

(12) # EUROPEAN PATENT SPECIFICATION

(45) Date of publication of patent specification: **02.05.91**

(51) Int. Cl.[5]: **C07D 471/04**, A61K 31/435, //(C07D471/04,239:00,221:00)

(21) Application number: **87305636.0**

(22) Date of filing: **24.06.87**

(54) **4(3H)-Oxo-5,6,7,8,-Tetrahydropyrido-(2,3-d)Pyrimidine derivatives.**

(30) Priority: **30.06.86 US 879935**
**20.04.87 US 40330**

(43) Date of publication of application:
**03.02.88 Bulletin 88/05**

(45) Publication of the grant of the patent:
**02.05.91 Bulletin 91/18**

(84) Designated Contracting States:
**AT BE CH DE ES FR GB GR IT LI LU NL SE**

(56) References cited:
**EP-A- 0 075 880**
**WO-A-86/05181**

(73) Proprietor: **THE TRUSTEES OF PRINCETON UNIVERSITY**
**P.O. Box 36**
**Princeton, NJ 08544(US)**

(72) Inventor: **Taylor, Edward C.**
**288 Western Way**
**Princeton New Jersey 08544(US)**
Inventor: **Beardsley, George Peter**
**Tonawanda Valley Box 192**
**Essex Connecticut 06426(US)**
Inventor: **Shih, Chuan**
**8521 East Scarsdale Drive**
**Indianapolis Indiana 46256(US)**
Inventor: **Hamby, James M.**
**3802 Ravens Crest Drive**
**Plainsboro New Jersey 08536(US)**

(74) Representative: **Jump, Timothy John Simon et al**
**F.J. Cleveland and Company 40-43 Chancery Lane**
**London WC2A 1JQ(GB)**

**EP 0 255 228 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The invention pertains to derivatives of N-[4-(N-[2-amino-4(3H)-oxo-5,6,7,8-tetrahydropyrido[2,3-d]-pyrimidin-6-ylmethyl]amino)benzoyl]-L-glutamic acid, which derivatives are antineoplastic agents, to their preparation and use, and to intermediates useful in their preparation.

The folic acid antimetabolites aminopterin and amethopterin (also known as I0-methylaminopterin or methotrexate) are antineoplastic agents. These compounds inhibit enzymatic conversions involving metabolic derivatives of folic acid. Amethopterin, for example, inhibits dihydrofolate reductase, an enzyme necessary for the regeneration of tetrahydrofolate from the dihydrofolate which is formed during the conversion of 2-deoxyuridylate to thymidylate by the enzyme thymidylate synthetase.

Other derivatives of folic acid and aminopterin have been synthesized and tested as anti-metabolites. Among these are compounds in which a methylene or methylidene group occupies a position in the molecule normally occupied by an imino or nitrilo group, respectively. These derivatives have varying degrees of antimetabolic activity. I0-Deazaaminopterin is highly active (Sirotak et al., Cancer Treat. Rep., I978, 62, I047) and 5-deazaaminopterin has activity similar to that of amethopterin (Taylor et al., J. Org. Chem., I983, 48, 4852). 8,I0-Dideazaaminopterin is reported to be active (U.S. Patent No. 4,460,59I) and 5,8,I0-trideazaaminopterin exhibits activity against mouse L1210 leukemia (Yan et al., J. Heterocycl. Chem. 1979, 16, 541). 10-Deazafolic acid, on the other hand, shows no significant activity (struck et al., J. Med. Chem., 1971, 14, 693) and 5-deazafolic acid is only weakly cytotoxic. 8,10-Dideazafolic acid is only marginally effective as a dihydrofolate reductase inhibitor (De Graw et al., "Chemistry and Biology of Pteridines", Elsevier, 1979, 229) and 5,8,10-trideazafolic acid also shows only marginal activity against mouse L1210 leukemia (Oatis et al. , J. Med. Chem. , 1977, 20, 1393). 5,10-Dideazaaminopterin and 5,10-dideaza-5,6,7,8-tetrahydroaminopterin, and the corresponding 5,I0-dideazafolic acid derivatives are reported by Taylor et al., J. Med. Chem., 28:7, 914 (1985).

In EP 0,075,880 there are disclosed analogs of folic acid which exhibit antifolate activity which are differentiated from this invention by the partial hydrogenation of the pyridogroups.

The invention pertains to (i) 4(3H)-oxo-5,6,7,8-tetrahydropyrido(2, 3-d)pyrimidines of the formula:

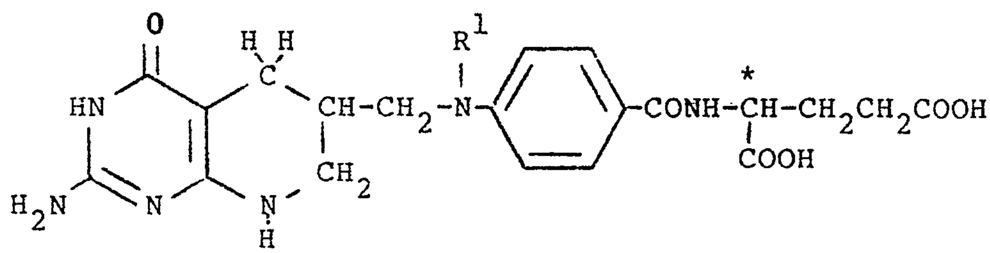

I

wherein:

$R^1$ is hydrogen, methyl, ethyl, or $R^5$ CO- in which $R^5$ is hydrogen or alkyl of 1 to 6 carbon atoms; and the configuration about the carbon atom designated * is L; (ii) the tautomeric forms thereof; and (iii) the pharmaceutically acceptable salts thereof.

The invention also pertains to methods for the preparation of such compounds, to intermediates useful in those preparations, and to methods and compositions for the use of such compounds in combating neoplastic growth.

The compounds of the invention are derivatives of the 5,6,7,8-tetrahydropyrido[2,3-d]pyrimidine heterocyclic ring which is numbered as follows:

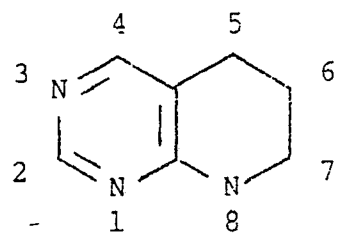

The compounds of Formula I exist in tautomeric equilibrium with the corresponding 4-hydroxy compounds:

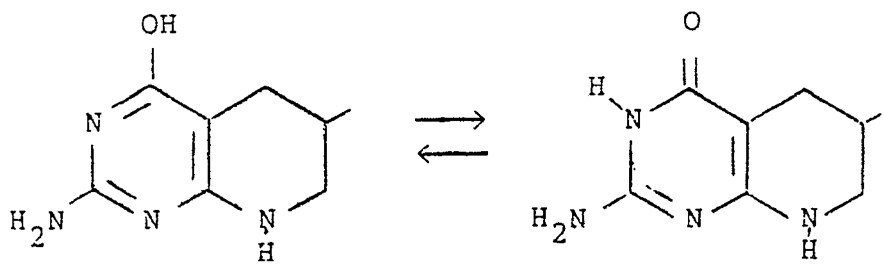

For convenience, the 4(3H)-oxo form is depicted and the corresponding nomenclature is used throughout this specification, it being understood that in each case such includes the tautomeric 3,4-dehydro-4-hydroxyform.

The absolute configuration about the carbon atom designated * in the glutamic acid chain is L, being the same absolute configuration as that about the corresponding alpha carbon atom in alanine. The carbon atom in the 6-position of the 5,6,7,8-tetrahydropyrido[2,3-d]pyrimidine ring system also is a chiral center, leading to d,L- and l,L-diastereoisomers. Both forms, which can be separated mechanically as by chromatography,are within the scope of the invention.

The invention includes the pharmaceutically acceptable salts. Salts with either acid (involving the nitrogen atom in the 8-position) or base (involving the carboxylic acid groups in the glutamic acid residue) can be formed. Those formed from base include the alkali metal, alkaline earth metal, non-toxic metal, ammonium, and substituted ammonium salts, such as for example the sodium, potassium, lithium, calcium, magnesium, aluminum, zinc, ammonium, trimethylammonium, triethylammonium, triethanolammonium, pyridinium, substituted pyridinium, and the like. Those formed from acid include pharmaceutically acceptable, non-toxic acid addition salts, such as are formed from (i) inorganic acids, as for example hydrochloric acid, hydrobromic acid, nitric acid, sulfuric acid, phosphoric acid, and the like (ii) organic carboxylic acids, as for example acetic acid, propionic acid, glycolic acid, succinic acid, maleic acid, hydroxymaleic acid, methylmaleic acid, fumaric acid, malic acid, tartaric acid, citric acid, benzoic acid, cinnamic acid, mandelic acid, salicylic acid, 4-aminosalicylic acid, 4-phenoxybenzoic acid, 2-acetoxybenzoic acid, embonic acid, nicotinic acid or isonicotinic acid, and (iii) organic sulfonic acids, as for example methansulfonic acid, ethanesulfonic acid, 2-hydroxyethanesulfonic acid, ethane-l,2-disulfonic acid, benzenesulfonic acid, p-toluenesulfonic acid or naphthalene-2-sulfonic acid.

The compounds of this invention have an effect on one or more enzymes which utilize folic acid, and in particular metabolic derivatives of folic acid, as a substrate.

The compounds can be prepared in a first process by hydrolysis or hydrogenolysis of a 2-amino-4(3H)-oxo-5,6,7,8-tetrahydropyrido[2,3-d]pyrimidinyl-L-glutamic acid derivative of the formula:

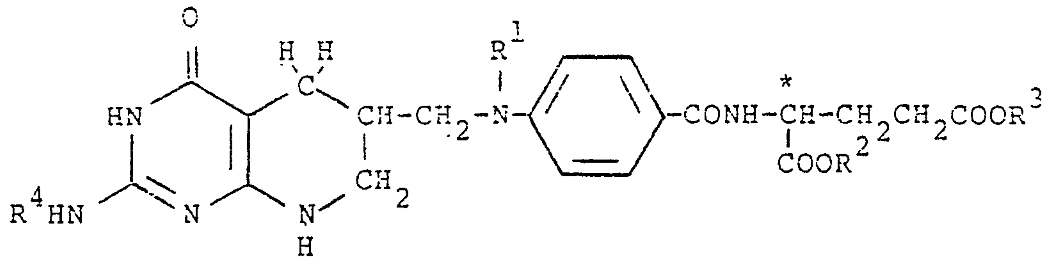

II

wherein

$R^1$ is as defined above;

$R^2$ and $R^3$ are the same or different carboxylic acid protecting group;

$R^4$ is an amino protecting group; and

the configuration about the carbon atom designated * is L.

Protecting groups encompassed by $R^2$, $R^3$ and $R^4$ and reactions for their removal are described, for example, in "Protective Groups in Organic Chemistry", Plenum Press, London and New York (l973); Greene, "Protective Groups in Organic Synthesis", Wiley, New York (1981); "The Peptides", Vol. I, Schröder and Lubke, Academic Press, London and New York (1965); in "Methoden der organischen Chemie", Houben-Weyl, 4th Edition, Vol. 15/I, Georg Thieme Verlag, Stuttgart (1974).

Carboxylic acid protecting groups can be, for example, esters conceptually derived from lower alkanols of from 1 to 6 carbon atoms, including those branched in the l-position and those which are substituted with one or more aromatic groups such as phenyl, or with halo or alkoxy; e.g., methyl, ethyl, t-butyl, benzyl, 4-nitrobenzyl, diphenylmethyl, methoxymethyl, and the like esters. Silyl esters such as trimethylsilyl also can be employed.

Amino protecting groups include acyl, notably alkanoyl of 2 to 6 carbon atoms, alkoxycarbonyl, each of which may be substituted with halo, alkoxy, or phenyl; e.g., acetyl, pivaloyl, 2,2,2-trichloroacetyl, benzoyl, t-butoxycarbonyl, 4-nitrobenzyloxycarbonyl, and the like.

The hydrolysis is conducted at normal temperatures utilizing dilute aqueous base, such as for example, 0.l-0.3 N aqueous alkali metal hydroxide, optionally in the presence of a water miscible organic solvent such as methanol, ethanol, tetrahydrofuran, dimethylformamide, and the like or an acid, as for example, trifluoroacetic acid. Use of acid or strong base will lead to hydrolysis if the -N'-$COR^5$ group is present. Some racemization of the glutamic acid portion of the molecule may be observed.

The product of the hydrolysis initially is formed as the dicationic glutamate salt and can be readily precipitated as the free acid by adjustment of pH through acidification with, for example, acetic acid or 0.5 N hydrochloric acid. The resulting products generally are high melting crystalline or microcrystalline solids.

The compounds of Formula I alternatively can be prepared, as can the glutamic acid intermediate of Formula II, by hydrogenating a pyrido[2,3-d]pyrimidine compound of the formula:

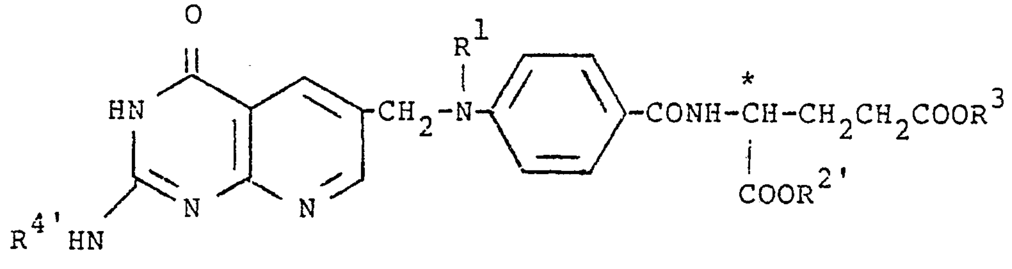

III

in which $R^1$ is as defined above; each of $R^{2'}$ , $R^{3'}$ is hydrogen or a carborylic acid protecting group as

defined above for $R^2$ and $R^3$ and $R^4$ is hydrogen or an amino protecting group as defined above for $R^4$. For example $R^{2'}$ and $R^{3'}$ may be ethyl and when this is the case $R^{4'}$ is either pivaloyl or acetyl.

The hydrogenation is conducted in an acidic medium in the presence of a noble metal catalyst such as platinum, ruthenium or rhodium, including the oxides thereof and the supported forms thereof. The preferred catalyst is platinum oxide. Conditions of time, temperature, and pressure are selected so that reduction of the pyridine ring is achieved without involvement of the pyrimidine ring. With platinum oxide, for example, the desired product is obtained in about l5 minutes utilizing ambient temperatures and a hydrogen pressure of 50 to 60 psi.

When $R^{2'}$, $R^{3'}$ and $R^{4'}$ are hydrogen, the product of this reduction will be a compound of Formula I. If all $R^{2'}$, $R^{3'}$ and $R^{4'}$ are other than hydrogen, the product will be a compound of Formula II.

Compounds of Formula III are known or can be prepared by conventional procedures. For example, 2-amino-4(3H)-oxo-6-formylpyrido[2,3-d]pyrimidine can be treated with an appropriate reagent to introduce the $R^4$ protecting group, such as acetic anhydride, and the resulting product allowed to react with a protected N-(4-aminobenzoyl)-L-glutamic acid derivative to yield the compound of Formula III herein in which $R^1$ = H [see e.g., Taylor et al., J. Org. Chem., 48, 4852 (l983)]. Utilization of the corresponding N-(4-methylaminobenzoyl)-L-glutamic acid or N-(4-ethylaminobenzoyl)-L-glutamic acid yields the corresponding compound of Formula III in which R′ is methyl or ethyl, respectively.

Compounds of Formula III in which $R^1$ is $R^5$CO-can be obtained by acylation of a compound of Formula III in which $R^1$ is hydrogen.

When $R^5$ is hydrogen, the acylation can be performed using an excess of formic acid [see e.g., Haynes et al, J. Med. Chem. 20, 588-59l (l977)] or a reactive acylating derivative of formic acid such as a mixed formic acid anhydride, as for example as is formed from formic acid and acetic anhydride.

When $R^5$ is alkyl, a reactive derivative of a carboxylic acid, as for example an acid halide such as acetyl chloride, can be employed.

The compounds of Formulas II and III in which $R^1$ is $R^5$CO- are valuable intermediates, particularly for the preparation of the final compound of Formula I in which $R^1$ is hydrogen, namely N-[4-(N′-[2-amino-4-(3H)-oxo-5,6,7,8-tetrahydropyrido[2,3-d]pyrimidin-6-ylmethyl]amino)benzoyl]-L-glutamic acid. This compound has antineoplastic activity and can be prepared through direct hydrogenation of a compound of Formula IV, optionally followed by removal of any protecting groups $R^{2'}$, $R^{3'}$ and $R^{4'}$. Introduction of an N′-acyl group, such as the formyl group, prior to reduction, however, affords added protection of the amino group which, being in the nature of a benzylic amine, is susceptible to hydrogenolysis. Thus by first forming the N′-formyl derivative of Formula III, hydrogenating the same to yield the N′-formyl-tetrahydro intermediate of Formula II, and then subjecting the same to hydrolysis, preferably acidic and basic hydrolysis performed sequentially, there is obtained N-[4-(N′-[2-amino-4(3H)-oxo-pyrido[2,3-d]pyrimidin-6-ylmethyl]amino)-benzoyl]-L-glutamic acid.

Two chiral centers are present in the final molecule: the carbon atom in the 6-position of the tetrahydropyrido[2,3-d]pyrimidine ring and the alpha oarbon atom in the glutamic acid group. Of the theoretical four forms of the compound, the use of a protected N-(4-aminobenzoyl)-L-glutamic acid reagent in the preparation of a compound of Formula III reduces the possibilities to two. Both of these, however, are generated during the subsequent hydrogenation to a compound of Formula II and consequently, upon removal of the protecting groups, the desired compound is produced as a mixture of the (S,S) and (R,S) diastereoisomers. These can be represented for the compound in which $R^{2'}$, $R^{3'}$ and $R^{4'}$ are all hydrogen as follows:

(S,S):

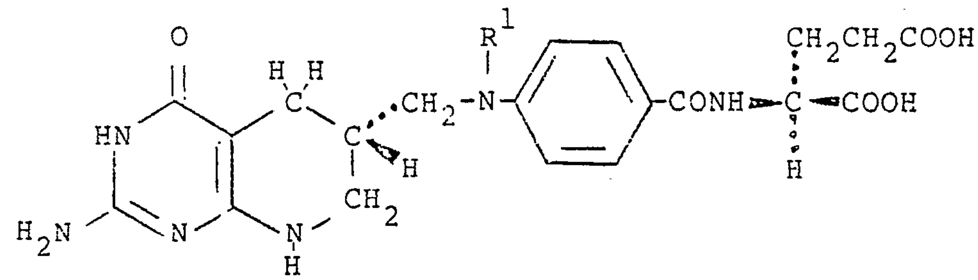

IV

(R,S):

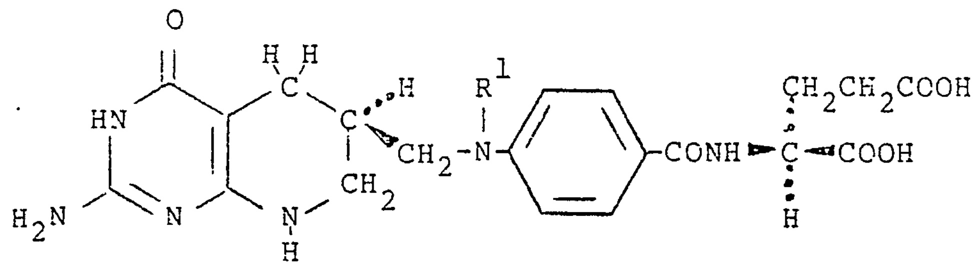

V

These diastereoisomers can be separated mechanically, as by chromatography, so that each is in a form substantially free of the other; i.e., having an optical purity of >95%. Alternatively, a mixture of diastereoisomeric compounds of Formula I is treated with a chiral acid operable to form a salt therewith. The resultant diastereoisomeric salts are then separated through one or more fractional crystallizations and thereafter the free base of the cationic moiety of at least one of the separated salts is liberated through treatment with a base and removal of the protecting groups. The liberation of the cation of the salt can be performed as a discrete step before or after the removal of the protecting groups, or concomitantly with the removal when such groups are susceptible to removal under basic conditions; i.e., basic hydrolysis.

Suitable chiral acids include the individual enantiomers of 10-camphorsulfonic acid, camphoric acid, alpha bromocamphoric acid, menthoxyacetic acid, tartaric acid, diacetyltartaric acid, malic acid, pyrrolidone-5-carboxylic acid, and the like.

The compounds of Formula I can be used, alone or in combination, to treat neoplasms which in the past have been treated with methotrexate, including chorio-carcinoma, leukemia, adenocarcinoma of the female breast, epidermid cancers of the head and neck, squamous or small-cell lung cancer, and various lymphosarcomas. In representative models for example, N-[4-(N'-[2-amino-4(3H)-oxo-5,6,7,8-tetrahydropyrido[2,3-d]pyrimidin-6-ylmethyl]amino)benzoyl]-L-glutamic acid exhibited $IC_{50}$ of 0.0052-0.0079 mcg/ml ($2.2 \times 10^{-7}$ molar) in the 72 hour assay against CCRF-CEM cell lines (a human T-cell derived leukemia). The diastereoisomers thereof exhibited $IC_{50}$'s of 0.0026 and 0.0027 mcg/ml, respectively. N-[4-(N'-[2-amino-4(3H)-oxo-5,6,7,8-tetrahydropyrido[2,3-d]pyrimidin-6-ylmethyl]-N'-formylamino)benzoyl]-L-glutamic acid exhibited an $IC_{50}$ of 0.011 in the same test. 5-Deazafolic acid on the other hand is relatively inactive in this test. The compounds can also be used to treat mycosis fungoides and psoriasis.

The compounds may be administered either orally or preferably parenterally, alone or in combination with other anti-neoplastic agents, steroids, etc., to a mammal suffering from neoplasm and in need of treatment. Parenteral routes of administration include intramuscular, intrathecal, intravenous or intra-arterial.

In general, the compounds are administered in much the same fashion as methotrexate, but because of a different mode of action, can be administered in higher dosages than those usually employed with methotrexate Leucovorin rescue is not needed. Dosage regimens must be titrated to the particular neoplasm, the condition of the patient, and the response but generally doses will be from about 10 to about

100 mg/day for 5-10 days or single daily administration of 250-500 mg, repeated periodically; e.g., every 14 days. Oral dosage forms include tablets and capsules containing from 1-10 mg of drug per unit dosage. Isotonic saline solutions containing 20-100 mg/ml can be used for parenteral administration.

The following examples will serve to further illustrate the invention.

Example 1

Diethyl N-[4-(N'-[2-acetamido-4(3H)-oxopyrido[2,3-d]pyrimidin-6-ylmethyl]amino)benzoyl]glutamate.

A mixture of 800 mg of 2-acetamido-6-formyl-4(3H)-oxopyrido[2,3-d]pyrimidine [Taylor et al. J. Org. Chem. 48, 4852 (1983)] in 55 ml of glacial acetic acid and 1.2 g of diethyl p-aminobenzoyl-L-glutamate is allowed to stand at room temperature for 5 hours. To the mixture is then added 0.19 ml of boron hydride:triethylamine complex. This mixture is stirred for 40 minutes at room temperature and then heated to 60°C for 10 minutes. The reaction mixture is cooled, and concentrated in vacuo. The resulting residue is dissolved in 90 ml of methanol, and the solution filtered. The solid which is collected is washed with 20 ml of methanol and 360 ml of ether. The filtrates are combined and evaporated to dryness. The residue is flash chromatographed [see Still et al., J. Org. Chem., 43, 2923 (1978)] over silica (97:3 chloroform:methanol) to yield 1.08 g of diethyl N-[4-(N'-[2-acetylamino-4(3H)-oxopyrido[2,3-d]pyrimidin-6-ylmethyl]amino)benzoyl]-L-glutamate.

Example 2

Diethyl N-[4-(N'-[2-acetamido-4(3H)-oxo-5,6,7,8-tetrahydropyrido[2,3-d]pyrimidin-6-ylmethyl]amino)benzoyl]-L-glutamate.

A mixture of 340 mg of diethyl N-[4-(N'-[2-acetylamino-4(3H)-oxopyrido[2,3-d]pyrimidin-6-ylmethyl]-amino)benzoyl]-L-glutamate in 80 ml of methanol and 40 ml of glacial acetic acid is placed in the vessel of a hydrogenation apparatus (Adams). Fifty-five milligrams of platinum oxide catalyst are added and the mixture is hydrogenated at 60 psi at room temperature for 15 minutes. The catalyst is removed by filtration, and the filtrate concentrated in vacuo. The residue is flash chromatographed over silica with chloroform:methanol gradients (97:3 to 95.5:5), collecting 20 ml fractions. Fractions 62-73 contain a by-product, 2-acetylamino-6-methyl-4(3H)-oxopyrido[2,3-d]pyrimidine. Fractions 74-88 contain 15.4 mg. of the desired product, diethyl N-[4-(N'-[2-acetyl-amino-4(3H)-oxo-5,6,7,8-tetrahydropyrido[2,3-d]pyrimidin-6ylmethyl]-amino)benzoyl]-L-glutamate.

Example 3

N-[4-(N'-[2-acetamido-4(3H)-oxo-5,6,7,8-tetrahydropyrido [2,3-d]pyrimidin-6-ylmethyl]amino)benzoyl]-L-glutamicacid.

Twenty milligrams of diethyl N-[4-(N'-[2-acetylamino-4(3H)-oxo-5,6,7,8-tetrahydropyrido[2,3-d]pyrimidin-6-ylmethyl]amino)benzoyl]-L-glutamate are dissolved in 8 ml of methanol and 0.4 ml of 1.0 N aqueous sodium hydroxide are added. The mixture is stirred at room temperature for 96 hours and 0.1 ml of glacial acetic acid is then added. The methanol is removed in vacuo and the resulting residue is dissolved in 5 ml of water. This mixture is acidified with 0.16 ml of glacial acetic and the solution which forms is collected by filtration to yield 6.0 mg. of product; N-[4-(N'-[2-acetamido-4(3H)-oxo-5,6,7,8-tetrahydropyrido[2,3-d]-pyrimidin-6-ylmethyl]amino)benzoyl]-L-glutamic acid, mass spectrum at 444.

Example 4

Diethyl N-[4-(N'-[2-acetamido-4(3H)-oxopyrido[2,3-d]pyrimidin-6-ylmethyl]-N'-formylamino)benzoyl]-glutamate.

A mixture of 98% formic acid (25 mL) and acetic anhydride (4.9 mL, 5.25 g, 51.5 mmol) is stirred at 25°C for 2 hours. To this solution is added 2.43 g (4.5 mmol) of diethyl N-[4-(N'-[2-acetamido-4(3H)-oxopyrido[2,3-d]pyrimidin-6-ylmethyl]amino)benzoyl]-L-glutamate [Taylor et al. J. Org. Chem. 48, 4852 (1983)]. This mixture is stirred at 55°C for 15 minutes, and at 25°C for 1 hour. The solvent is removed under reduced pressure and the residue triturated with ether. The solid is filtered and recrystallized from 2-propanol to give 2.0 g (78%) of diethyl N-[4-(N'-[2-acetamido-4(3H)-oxopyrido[2,3-d]pyrimidin-6-ylmethyl]N'-formylamino)benzoyl]glutamate: mp 180-182°C; $^1H$ NMR ($Me_2SO$-$d_6$) delta 1.05-1.23 (m, 6 H), 1.95-2.10 (m, 2H), 2.16 (s, 3H), 2.49 (t, 1H, J = 7.36 Hz), 3.97-4.10 (m, 4H), 4.36-4.39 (m, 1H), 5.24 (m, 2H), 7.51-7.54 (m, 2H, AA'BB'), 7.84-7.87 (m, 2H, AA'BB'), 8.21 (m, 1H), 8.69-8.75 (m, 2H), 8.82 (s, 1H).
Anal. Calcd. for $C_{27}H_{30}N_6O_8$: C, 57.24; H, 5.34; N, 14.83.
Found: C, 56.94; H, 5.11; N, 14.57.

Example 5

Diethyl N-[4-(N'-[2-pivaloylamino-4(3H)-oxopyrido[2,3-d]pyrimidin-6-ylmethyl]-N'-formylamino)benzoyl]-glutamate.

The title compound is prepared analogously to the procedure of Example 4 utilizing diethyl N-[4-(N'-[2-pivaloylamino-4(3H)-oxopyrido[2,3-d]pyrimidin-6-ylmethyl]amino)benzoyl]-L-glutamate; $^1H$ NMR ($Me_2SO$-$d_6$) delta 1.09-1.16 (m, 6 H), 1.21 (s, 9H), 1.90-2.10 (m, 2H), 2.39 (t, 2H, J = 7.37 Hz), 3.96-4.09 (m, 4H), 4.31-4.43 (m, 1H), 5.22 (s, 2H), 7.50-7.53 (AA'BB', 2H), 7.83-7.86 (AA'BB'), 8.21 (m, 1H), 8.71 (d, 1H, J = 5.87 Hz), 8.72 (m, 1H), 8.80 (s, 1H).

Example 6

Diethyl N-[4-(N'-[2-pivaloylamino-4(3H)-oxopyrido[2,3-d]pyrimidin-6-ylmethyl]-N'-acetylamino)benzoyl]-glutamate.

To a stirred suspension of 0.65 g of diethyl N-[4-(N'-[2-pivaloylamino-4(3H)-oxopyrido[2,3-d]pyrimidin-6-ylmethyl]-amino)benzoyl]glutamate (1.1 mmol) and 0.22 g (2.2 mmols) of potassium bicarbonate in 10mL of methylene chloride cooled to 0-5°C is added 0.097 g (1.2 mmol) of acetyl chloride. The reaction mixture is stirred at 0-5°C for 10 minutes and then allowed to attain room temperature. After stirring at ambient temperature for 45 minutes, 50 mL of methylene chloride are added. The mixture is extracted with 25 mL of water, 25 mL of a saturated solution of sodium bicarbonate, and again with 25 mL of water. The aqueous extracts are combined and back-extracted with 50 mL of methylene chloride. The organic extracts are dried over anhydrous magnesium sulfate, and filtered, and the solvent is removed under reduced pressure to give 0.7 g of diethyl N-[4-(N'-[2-pivaloylamino-4(3H)-oxopyrido[2,3-d] pyrimidin-6-ylmethyl]-N'-acetylamino)-benzoyl]glutamate; $^1H$ NMR ($Me_2SO$-$d_6$) delta 1.10-1.17 (m, 6 H), 1.22 (s, 9H), 1.88 (s, 3 H), 1.88-2.13 (m, 2H), 2.40 (t, 2H, J = 7.46 Hz), 3.97-4.08 (m, 4H), 4.30-4.42 m, 1H), 5.02 (s, 2H), 7.34-7.36 (AA'BB', 2H), 7.83-7.86 (AA'BB', 2H), 8.19 (m, 1H), 8.64 (m, 1H), 8.75 (d, 1H, J = 7.39 Hz).

Example 7

Diethyl N'-[4-(N-[2-acetamido-4(3H)-oxo-5,6,7,8-tetrahydropyrido[2,3-d]pyrimidin-6-ylmethyl]-N'-formylamino) benzoyl]-L-glutamate.

A mixture of 0.743 g (0.36 mmol) of diethyl N-[4-(N'-[2-acetamido-4(3H)-oxopyrido[2,3-d]pyrimidin6-ylmethyl]-N'-formylamino)benzoyl]-L-glutamate and 230 mg of platinum oxide in 75 mL of a 2:1 ethanol:acetic acid solution is shaken under an atmosphere of hydrogen (40 psi) for 1.5 hours. The reaction

mixture is filtered through Celite and the filtrate evaporated first under aspirator vacuum and then under high vacuum, maintaining the temperature as low as possible. The residue is dissolved in methylene chloride and chromatographed (Chromatotron) eluting with 5% methanol in methylene chloride to give 0.684 g (93%) of diethyl N-[4-(N'-[2-acetamido-4(3H)-oxo-5,6,7,8-tetrahydropyrido[2,3-d]pyrimidin-6-ylmethyl]-N'-formylamino)benzoyl]-L-glutamate; mp 188-190 °C; $^1$H NMR ($Me_2SO-d_6$)delta 1.12-1.19 (M, 6 H, ester $CH_3$'s), 1.89-2.11 (m, 4H), 2.08 (s, 3H), 2.42 (t, 2H, J = 7.26 Hz), 2.78-2.91 (m, 1H), 3.09-3.19 (m, 1 H), 3.89 (d, 2H, J = 5.98 Hz), 3.99-4.12 (m, 4H), 4.42 (m, 1H), 6.64 (m, 1H), 7.51-7.54 (m, 2H, AA'BB'), 7.91-7.94 (m, 2H, AA'BB'), 8.61 (s, 1H), 8.74 (d, 1H, J = 7.32 Hz).

An analytical sample is prepared by chromatographing a portion of the above sample taking a center fraction. After removal of the solvent the sample was triturated with ether and the solid collected.
Anal. Calcd. for $C_{27}H_{34}N_6O_8$ C,56.83; H, 6.01; N, 14.73.
Found: C, 56.60; H, 5.90; N, 14.43.

## Example 8

Diethyl N-[4-(N'-[2-pivaloylamino-4(3H)-oxo-5,6,7,8-tetrahydropyrido[2,3-d]pyrimidin-6-ylmethyl]-N'-formylamino)benzoyl]-L-glutamate.

Diethyl N-[4-(N'-[2-pivaloylamino-4(3H)-oxo-5,6,7,8-tetrahydropyrido[2,3-d]pyrimidin-6-ylmethyl]-N'-formylamino)benzoyl]-L-glutamate is similarly obtained upon reduction of diethyl N-[4-(N'-[2-pivaloylamino-4-(3H)-oxopyrido[2,3-d]pyrimidin-6-ylmethyl]-N'-formylamino)benzoyl]glutamate; mp 152-153 °C; IR (KBr) $V_{max}$ 3369 and 3250.(NH), 1732, 1637, and 1605 (C=O) $cm^{-1}$; $^1$H NMR ($Me_2SO-d_6$) delta 1.12-1.2 (m, 6 H), 1.17 (s, 9H), 1.90-2.18 (m, 4H), 2.43 (t, 2H, J = 7.4 Hz), 2.80-2.94 (m, 1H), 3.12-3.20 (m, 1 H), 3.89 (d, 2H, J = 5.59 Hz), 4.00-4.11 (m, 4H), 3.90-4.47 (m, 1H), 6.40 (m, 1H), 7.52-7.55 (AA'BB', 2H), 7.92-7.94 (AA'BB', 2H), 8.62 (s, 1H), 8.75 (d, 1H, J = 7.33 Hz).
Anal. Calcd. for $C_{30}H_{40}N_6O_6$ C,58.81; H, 6.58; N, 13.72.
Found: C, 58.53; H, 6.60; N, 13.61.

## Example 9

Diethyl N-[4-(N'-[2-pivaloylamino-4(3H)-oxo-5,6,7,8-tetrahydropyrido[2,3-d]pyrimidin-6-ylmethyl]-N'-acetylamino)benzoyl]-L-glutamate.

To a solution of 0.64 g (1.03 mmol) of diethyl N-[4-(N'-[2-pivaloylamino-4(3H)-oxopyrido[2,3-d]pyrimidin-6-ylmethyl]-N'-acetylamino)benzoyl]-L-glutamate in 40 mL of glacial acetic acid are added 96 mg of platinum oxide. The suspension is shaken under an atmosphere of hydrogen (45 psi) for 2.5 hours, mixture diluted with 100 mL of methylene chloride and filtered through Celite to remove the catalyst. The filtrate is evaporated under reduced pressure and the residue is dissolved in 100 mL of methylene chloride, and extracted twice with 75 mL portions of a saturated solution of sodium bicarbonate. The aqueous layers are back-extracted with 75 mL of methylene chloride and the organic layers are combined and dried over anhydrous magnesium sulfate. After removing the drying agent by filtration, the solvent is removed from the filtrate under reduced pressure to give 0.54 g (84% yield) of diethyl N-[4-(N'-[2-pivaloylamino-4(3H)-oxo-5,6,7,8-tetrahydropyrido[2,3-d]pyrimidin-6-ylmethyl]-N'-acetylamino)benzoyl]-L-glutamate which can be further purified by recrystallization from ethyl acetate; mp 120-123 °C $^1$H NMR ($Me_2SO-d_6$) delta 1.06-1.19 (m, 6 H), 1.16 (s, 9H), 1.82 (s, 3H) 1.84-2.17 (m, 6H), 2.42 (t, 2H, J = 7.40 Hz), 2.80-2.94 (m, 1H), 3.17-3.23 (m, 1 H), 3.68 (d, 2H, J = 5.59 Hz), 3.98-4.10 (m, 4H), 4.39-4.45 (m, 1H), 6.38 (s, 1H), 7.43-7.46 (AA'BB', 2H), 7.90-7.93 (AA'BB', 2H), 8.79 (d, 1H, J = 7.33 Hz).

## Example 10

N-[4-(N'-[2-amino-4(3H)-oxo-5,6,7,8-tetrahydropyrido[2,3-d]pyrimidin-6-ylmethyl]-N'-formylamino)benzoyl]-L-glutamic acid.

A mixture of 0.092 g (0.l6 mmol) of diethyl N-[4-(N'-[2-acetamido-4(3H)-oxo-5,6,7,8-tetrahydropyrido[2,3-d]pyrimidin-6-ylmethyl]-N'-formylamino)benzoyl]-L-glutamate in l0 mL of a 0.25 aqueous sodium hydroxide solution is stirred at 25° C for 72 hours and then water evaporated under reduced pressure. The residue is dissolved in l5 mL of water, and the solution is cooled to 0° C and acidified with acetic acid. The solid is collected after 30 minutes to give 0.046 g (60.5%) of N-[4-(N'-[2-amino-4(3H)-oxo-5,6,7,8-tetrahydropyrido [2,3-d]pyrimidin-6-ylmethyl]-N'-formylamino)benzoyl]-L-glutamic acid, $^{1}H$ NMR ($Me_2SO-d_6$) delta l.8l-2.2 (m, 4H), 2.33 (m, 3H), 2.73 (m, lH), 3.l (m, lH), 3.85 (m, 2H), 4.39 (m, lH), 6.2 (m, lH), 7.49-7.5l 5 (m, 2H, AA'BB'), 7..90-7.93 (m, 2H, AA'BB'), 8.59-8.62 (m, 2H).

Analogously, 200 mg of diethyl N'-[4-(N-[2-pivaloylamino-4(3H)-oxo-5,6,7,8-tetrahydropyrido[2,3-d]-pyrimidin-6-ylmethyl]-N'-acetylamino)benzoyl]-L-glutamate is stirred for 72 hours in 5 mL of 0.2 N sodium hydroxide. The reaction mixture is neutralized with 0.5 hydrochloric acid, cooled to 0° C and the solid which forms is collected by filtration to yield N-[4-(N'-[2-amino-4(3H)-oxo-5,6,7,8-tetrahydropyrido[2,3-d]pyrimidin-6-ylmethyl]-N'-acetylamino)benzoyl]-L-glutamic acid.

## Example ll

N-[4-(N'-[2-amino-4(3H)-oxo-5,6,7,8-tetrahydropyrido[2,3-d]pyrimidin-6-ylmethyl]-N'-formylamino)benzoyl]-L-glutamic acid.

A solution of 20 mg of N-[4-(N'-[2-amino-4(3H)-oxo-5,6,7,8-tetrahydropyrido[2,3-d]pyrimidin-6-ylmethyl]-amino)benzoyl]-L-glutamic acid in 0.5 mL of 97% formic acid is heated at 90° C for l hour. The solvent is removed under reduced pressure and the residue triturated with ether. The insoluble solid is collected to give l7 mg of product, N-[4-(N'-[2-amino-4(3H)-oxo-5,6,7,8-tetrahydropyrido[2,3-d]pyrimidin-6-ylmethyl]-N'-formylamino)benzoyl]-L-glutamic acid, which is further purified by dissolution in 0.l N sodium hydroxide and precipitation through the addition of glacial acetic acid.

## Example l2

N-[4-(N'-[2-amino-4(3H)-oxo-5,6,7,8-tetrahydropyrido[2,3-d]pyrimidin-6-ylmethyl]amino)benzoyl]-L-glutamic acid.

To 32 mL of a 5% of hydrochloric acid in methanol solution (prepared by diluting 2 mL of concentrated hydrochloric acid to 60 mL with methanol) is added 0.7l7 g (l.3 mmol) of diethyl N-[4-(N'-[2-acetamido-4-(3H)-oxo-5,6,7,8-tetrahydropyrido[2,3-d]pyrimidin-6-ylmethyl]-N'-formylamino)benzoyl]-L-glutamate (prepared as in Example 2). The reaction mixture is stirred at 45° C for l8 hours. After allowing the reaction mixture to cool at 25° C, 4 mL of sodium hydroxide (6 N) are added and the mixture stirred for another 72 hours at 25° C. The solution is concentrated under reduced pressure. Twenty milliliters of water are added, and the mixture acidified by adding glacial acetic acid dropwise. After standing 2 hours at 0° C, the solid is collected to give 0.485 g (88% yield) of N-[4-(N'-[2-amino-4(3H)-oxo-5,6,7,8-tetrahydropyrido[2,3-d]-pyrimidin-6-ylmethyl]amino)benzoyl]-L-glutamic acid; mp decomposition beginning at l98° C; $^{1}H$ NMR ($Me_2SO-d_6$) delta l.86-2.l (m, 6H), 2.3l (t, 2H, J=7.2), 2.8-2.86 (m, lH), 3.24-3.28 (m, 2H), 4.2-4.4 (m, lH), 5.94 (s, 2H), 6.29 (s, lH), 6.34 (t, lH, J = 5.24), 6.56-6.58 (AA'BB', 2H), 7.62-7.65 (AA'BB', 2H), 8.06 (d, J = 5.l5), 9.7 (br, s, lH).

The same product can be obtained through hydrolysis of diethyl N-[4-(N'-[2-pivaloylamino-4(3H)-oxopyrido[2,3-d]pyrimidin-6-ylmethyl]-N'-formylamino)benzoyl]glutamate.

Alternatively, l.44 g (2.4 mmols) of diethyl N'-[4-(N-[2-pivaloylamino-4(3H)-oxo-5,6,7,8-tetrahydropyrido-[2,3-d]pyrimidin-6-ylmethyl]-N'-formylamino)benzoyl]-L-glutamate is dissolved in lN sodium hydroxide and the solution is stirred at 25° C for 72 hours. Charcoal is added and the suspension is stirred and filtered. The filtrate is acidified with glacial acetic acid and the white solid is collected after 30 minutes cooling at 0° C to yield 0.87 g (84% yield) of the same product; mp slow decomposition over l98° C; IR (KBr) $V_{max}$ 3460-2500 (NH and COOH), l695, l655, and l600 (C=O) $cm^{-1}$; $^{1}H$ NMR ($Me_2SO-d_6$) delta l.85-2.02 (m, 6 H), 2.3 (t, 2H, J = 7.4 Hz), 2.8l-2.88 (m, lH) 3.23-3.32 (m, 2H) 4.2-4.4 (m, lH), 5.92 (s, 2H), 6.34 (t, lH, J = 5.28 Hz), 6.55-6.57 (m, 2H AA'BB'), 7.6l-7.64 (AA'BB', 2H), 8.08 (d, lH, J = 7.64 Hz), 9.7 (br, s, lH).

Example I3

A I mg/ml solution of N-[4-(N'-[2-amino-4(3H)-oxo-5,6,7,8-tetrahydropyrido[2,3-d]pyrimidin-6-ylmethyl]-amino)benzoyl]-L-glutamic acid in I5% acetonitrile and 85% of a 0.I% solution of triethylamineacetic acid (buffered to pH 7.0) is prepared. This solution is introduced into a I0 mm x 50 cm Cyclobond I reverse phase HPLC column utilizing the same solvent system. A flow rate of I.I0 ml/min is employed with UV monitoring at 254 nm.

A first diastereoisomer substantially free of the other is obtained at a retention time of 45.58 minutes, herein designated Isomer "A". The second diastereoisomer substantially free of the first is obtained at a retention time of 48.32 minutes, herein designated Isomer "B".

Example I4

Groups of I0 C3H female mice were innoculated intraperitoneally in the axillary region with C3H mammary adenocarcinoma cells. The test compound is then administered in Emulphor IP (0.5 mL) for I0 days. Controls received identical treatment without test compound.

N-[4-(N'-[2-amino-4(3H)-oxo-5,6,7,8-tetrahydropyrido[2,3-d]pyrimidin-6-ylmethyl]amino)benzoyl]-L-glutamic acid produced 96% inhibition with 7 out of I0 survivors at a dose of 6.25 mg/kg.

Against 6C3HED lymphosarcoma with an 8 day period of treatment, the following results were observed for the same compound:

| Dose (mg/kg) | Inhibition | Survivors |
|---|---|---|
| 6.0 | 77 | 10/10 |
| 12.5 | 100 | 9/10 |
| 25.0 | 100 | 7/10 |
| 50.0 | 100 | 4/10 |
| 100.0 | 100 | 1/10 |

## Claims

1. A compound selected from the group consisting of:

(i) 4(3H)-oxo-5,6,7,8-tetrahydropyrido[2,3-d] pyrimidines of the formula:

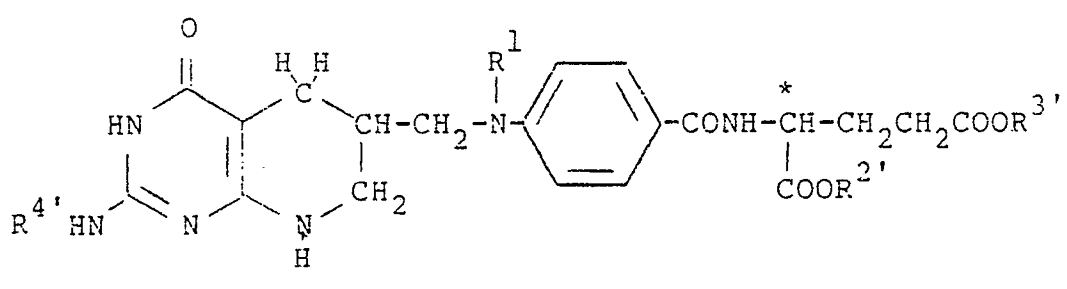

wherein

$R^1$ is hydrogen, methyl, ethyl, or $R^5CO$-;

$R^{2'}$ and $R^{3'}$ are hydrogen or the same or different carboxylic acid protecting group;

$R^{4'}$ is hydrogen or an amino protecting group; and

$R^5$ is hydrogen or alkyl of l to 6 carbon atoms; and the configuration about the carbon atom designated * is L; and

(ii) the tautomeric forms thereof.

**2.** A compound according to claim l wherein $R^1$ is hydrogen, each of $R^{2'}$ and $R^{3'}$ is hydrogen or alkyl of l to 6 carbon atoms and $R^{4'}$ is hydrogen or alkanoyl of 2 to 6 carbon atoms.

**3.** The compound according to claim 2 in which each of is acetyl.

**4.** The compound according to claim 2 in which each of $R^{2'}$ and $R^{3'}$ is ethyl, and $R^{4'}$ is pivaloyl.

**5.** The compound according to claim 2 wherein each of $R^{2'}$, $R^{3'}$ and $R^{4'}$ is hydrogen.

**6.** The R,S-diastereoisomer of the compound according to claim 5.

**7.** the S,S-diastereoisomer of the compound according to claim 5.

**8.** A compound according to claim 1 wherein $R^1$ is formyl, each of $R^{2'}$ and $R^{3'}$ is hydrogen or alkyl of 1 to 6 carbon atoms and $R^{4'}$ is hydrogen or alkanoyl of 2 to 6 carbon atoms.

**9.** The compound according to claim 8 in which each of $R^{2'}$ and $R^{3'}$ is ethyl, and $R^{4'}$ is acetyl.

**10.** The compound according to claim 8 in which each of $R^{2'}$ and $R^{3'}$ is ethyl, and $R^{4'}$ is pivaloyl.

**11.** The compound according to claim 8 wherein each of $R^{2'}$, $R^{3'}$, and $R^{4'}$ is hydrogen.

**12.** The R,S-diastereoisomer of the compound according to claim 11.

**13.** The S,S-diastereoisomer of the compound according to claim 11.

**14.** A compound according to claim 1 wherein $R^1$ is acetyl, each of $R^{2'}$ and $R^{3'}$ is hydrogen or alkyl of 1 to 6 carbon atoms and $R^{4'}$ is hydrogen or alkanoyl of 2 to 6 carbon atoms.

**15.** The compound according to claim 14 in which each of $R^{2'}$ and $R^{3'}$ is ethyl, and $R^{4'}$ is acetyl.

**16.** The compound according to claim 14 in which each of $R^{2'}$ and $R^{3'}$ is ethyl, and $R^{4'}$ is pivaloyl.

**17.** The compound according to claim 14 in which each of $R^{2'}$, $R^{3'}$ and $R^{4'}$ is hydrogen.

**18.** A pharmaceutical composition for combating neoplastic growth in a mammal which comprises an amount of a compound according to any one of the preceding claims which upon administration to the mammal in a single or multiple dose regimen is effective to combat said growth, in combination with a pharmaceutically acceptable carrier.

**19.** A pharmaceutical composition for combating neoplastic growth in a mammal which comprises an amount of a compound according to any of claims 5-7 or 11-13 which upon administration to the mammal in a single or multiple dose regimen is effective to combat said growth, in combination with a pharmaceutically acceptable carrier.

**20.** A process for the preparation of a compound of the formula:

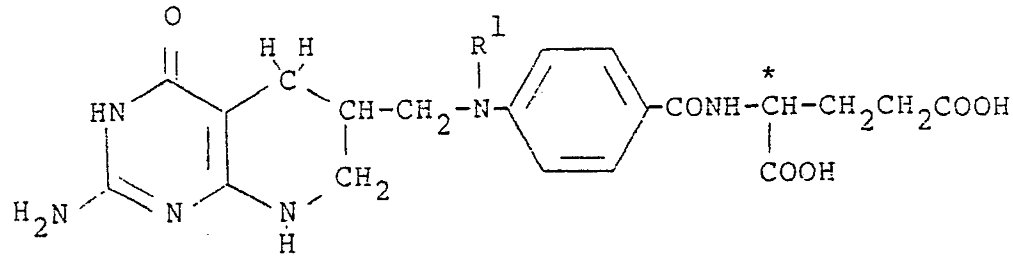

wherein:
$R^1$ is hydrogen or $R^5CO$-;
$R^5$ is hydrogen or alkyl of 1 to 6 carbon atoms; and
the configuration about the carbon atom designated * is L, and the tautomeric forms thereof, which comprises hydrolysing a compound of the formula:

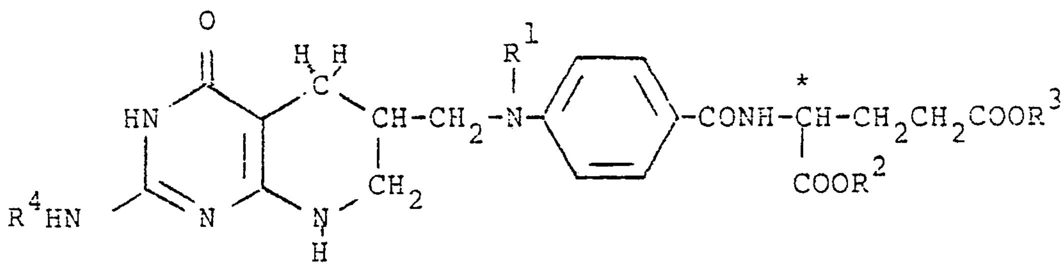

wherein
$R^1$ is as herein defined;
$R^2$ and $R^3$ are the same or different carboxylic acid protecting group; and
$R^4$ is an amino protecting group.

**21.** The process according to claim 20 wherein $R^1$ is hydrogen, each of $R^2$ and $R^3$ is alkyl of I to 6 carbon atoms and $R^4$ is alkanoyl of 2 to 6 carbon atoms.

**22.** The process according to claim 2I in which each of $R^2$ and $R^3$ is ethyl, and $R^4$ is acetyl.

**23.** The process according to claim 2I in which each of $R^2$ and $R^3$ is ethyl, and $R^4$ is pivaloyl.

**24.** The process according to claim 20 wherein $R^1$ is formyl, each of $R^2$ and $R^3$ is alkyl of I to 6 carbon atoms and $R^4$ is alkanoyl of 2 to 6 carbon atoms.

**25.** The process according to claim 24 in which each of $R^2$ and $R^3$ is ethyl, and $R^4$ is acetyl.

**26.** The process according to claim 24 in which each of $R^2$ and $R^3$ is ethyl, and $R^4$ is pivaloyl.

**27.** The process according to claim 20 wherein $R^1$ is acetyl, each of $R^2$ and $R^3$ is alkyl of I to 6 carbon atoms and $R^4$ is alkanoyl of 2 to 6 carbon atoms.

**28.** The process according to claim 27 in which each of $R^2$ and $R^3$ is ethyl, and $R^4$ s acetyl.

**29.** The process according to claim 27 in which each of $R^2$ and $R^3$ is ethyl, and $R^4$ is pivaloyl.

**30.** The process for the preparation of a compound according to claim I in which $R^1$ is $R^5CO$- which comprises treating a compound of the formula:

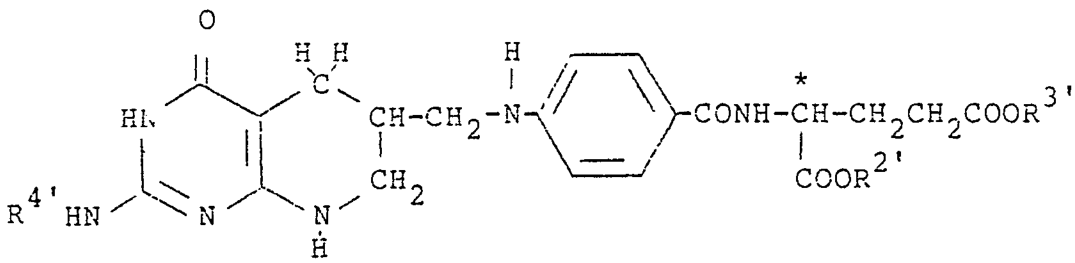

wherein

$R^{2'}$ and $R^{3'}$ are hydrogen or the same or different carboxylic acid protecting group; and

$R^{4'}$ is hydrogen or an amino protecting group; and

the configuration about the carbon atom designated * is L,

with an acid of the formula $R^5COOH$ in which $R^5$ is hydrogen or alkyl of I to 6 carbon atoms, or a reactive acylating derivative thereof.

**31.** The process according to claim 30 wherein each of $R^{2'}$, $R^{3'}$, $R^{4'}$ and $R^5$ is hydrogen.

**32.** The process according to claim 3I wherein each of $R^{2'}$ and $R^{3'}$ is alkyl of I to 6 carbon atoms, $R^{4'}$ is alkanoyl of 2 to 6 carbon atoms and $R^5$ is hydrogen.

**33.** The process for the preparation of a compound according to claim I which comprises catalytically hydrogenation of a compound of the formula:

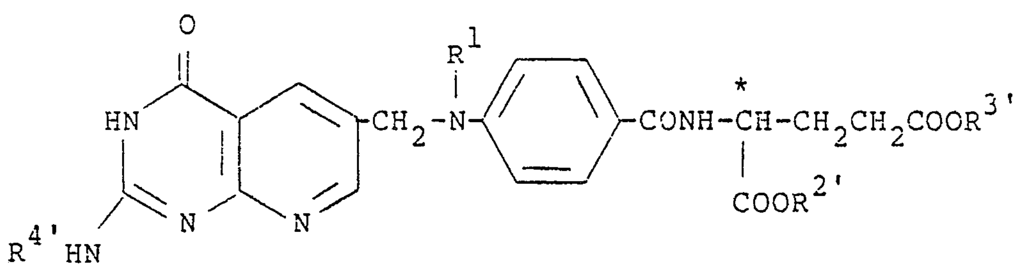

wherein $R^1$, $R^{2'}$, $R^{3'}$ $R^{4'}$ are as therein defined.

**34.** The process according to claim 33 wherein $R^1$ is hydrogen, each of $R^{2'}$ and $R^{3'}$ is alkyl of I to 6 carbon atoms and $R^{4'}$ is alkanoyl of 2 to 6 carbon atoms.

**35.** The process according to claim 34 in which each of $R^{2'}$ and $R^{3'}$ is ethyl, and $R^{4'}$ is acetyl.

**36.** The process according to claim 34 in which each of $R^{2'}$ and $R^{3'}$ is ethyl, and $R^{4'}$ is pivaloyl.

**37.** The process according to claim 33 wherein each of $R^{2'}$, $R^{3'}$, and $R^{4'}$ is hydrogen.

**38.** The process according to claim 33 wherein $R^1$ is formyl, each of $R^{2'}$ and $R^{3'}$ is alkyl of I to 6 carbon atoms, and $R^{4'}$ is alkanoyl of 2 to 6 carbon atoms.

**39.** The process according to claim 38 in which each of $R^{2'}$ and $R^{3'}$ is ethyl, and $R^{4'}$ is acetyl.

**40.** The process according to claim 38 in which each of $R^{2'}$ and $R^{3'}$ is ethyl, and $R^{4'}$ is pivaloyl.

**41.** The process according to claim 33 wherein $R^1$ is formyl and each of $R^{2'}$, $R^{3'}$, and $R^{4'}$ is hydrogen.

**Revendications**

**1.** Composé choisi dans le groupe constitué par :

(i) les 4(3H)-oxo-5,6,7,8-tétrahydropyrido-[2,3-d]pyrimidines de formule :

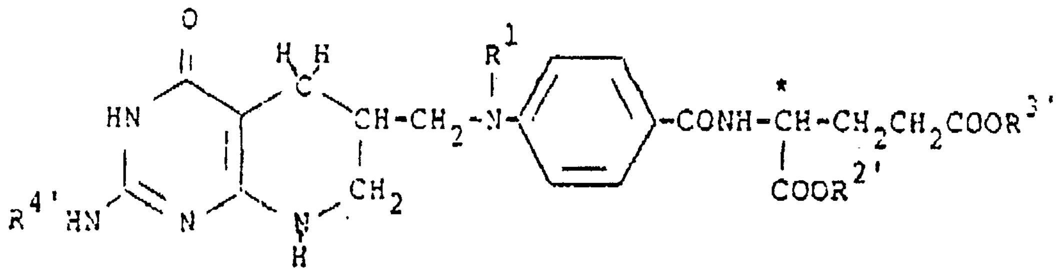

où

$R^1$ est un hydrogène, méthyle, éthyle, ou $R^5CO$- ;

$R^{2'}$ et $R^{3'}$ représentent un hydrogène ou un groupe protecteur d'acide carboxylique identique ou différent ;

$R^{4'}$ est un hydrogène ou un groupe Protecteur d'amino ; et

$R^5$ est un hydrogène ou un alcoyle en $C_{1-6'}$ et la configuration autour de l'atome de carbone est désignée par * est L ; et

(ii) leurs formes tautomériques.

**2.** composé selon la revendication 1, dans lequel $R^1$ est un hydrogène, chacun des radicaux $R^{2'}$ et $R^{3'}$ est un hydrogène ou un alcoyle en $C_{1-6}$ et $R^{4'}$ est un hydrogène ou un alcanoyle en $C_{2-6}$.

**3.** Composé selon la revendication 2, dans Lequel chacun des radicaux $R^{2'}$ et $R^{3'}$ est un éthyle, et $R^{4'}$ est un acétyle.

**4.** Composé selon la revendication 2, dans lequel chacun des radicaux $R^{2'}$ et $R^{3'}$ est un éthyle, et $R^{4'}$ est un pivaloyle.

**5.** Composé selon la revendication 2, dans lequel chacun des radicaux $R^{2'}$, $R^{3'}$ et $R^{4'}$ est un hydrogène.

**6.** Le diastéréoisomère R,S du composé selon la revendication 5.

**7.** Le diastéréoisomère S,S du composé selon la revendication 5.

**8.** Composé selon la revendication 1, dans lequel $R^1$ est un formyle, chacun des radicaux $R^{2'}$ et $R^{3'}$ est un hydrogène ou un alcoyle en $C_{1-6}$. et $R^{4'}$ est un hydrogène ou un alcanoyle en $C_{2-6}$.

**9.** Composé selon la revendication 8, dans lequel chacun des radicaux $R^{2'}$ et $R^{3'}$ est un éthyle, et $R^{4'}$ est un acétyle.

**10.** Composé selon la revendication 8, dans lequel chacun des radicaux $R^{2'}$ et $R^{3'}$ est un éthyle et $R^{4'}$ est un pivaloyle.

**11.** Composé selon la revendication 8, dans lequel chacun des radicaux $R^{2'}$ et $R^{3'}$ et $R^{4'}$ est un hydrogène.

**12.** Le diastéréoisomère R,S du composé selon la revendication 11.

**13.** Le diastéréoisomère S,S du composé selon la revendication 11.

**14.** Composé selon la revendication 1, dans lequel $R^1$ est un acétyle, chacun des radicaux $R^{2'}$ et $R^{3'}$ est un hydrogène ou un alcoyle en $C_{1-6}$ et $R^{4'}$ est un hydrogène ou un alcanoyle en $c_{2-6}$.

**15.** Composé selon la revendication 14, dans lequel chacun des radicaux $R^{2'}$ et $R^{3'}$ est un éthyle, et $R^{4'}$ est un pivaloyle.

**16.** Composé selon la revendication 14, dans lequel chacun des radicaux $R^{2'}$ et $R^{3'}$ est un éthyle, et $R^{4'}$ est un acétyle.

**17.** composé selon la revendication 14, dans lequel chacun des radicaux $R^{2'}$, $R^{3'}$ et $R^{4'}$ est un hydrogène.

**18.** Composition pharmaceutique pour combattre la croissance néoplasique chez un mammifère comprenant une quantité d'un composé selon l'une quelconque des revendications précédentes, qui lorsqu'on l'administre aux mammifères dans un régime posologique à une seule dose ou à doses multiples, est officace pour combattre ladite croissance, en combinaison avec un support pharmaceutiquement acceptable.

**19.** Composition pharmaceutique pour combattre la croissance néoplasique chez un mammifère qui comprend une quantité d'un composé selon l'une quelconque des revendications 5-7 ou 11-13, qui, après administration au mammifère en un régime posologique à une seule dose ou à doses multiples, est efficace pour combattre ladite croissance, en combinaison avec un support pharmaceutiquement acceptable.

**20.** procédé de préparation d'un composé de formule :

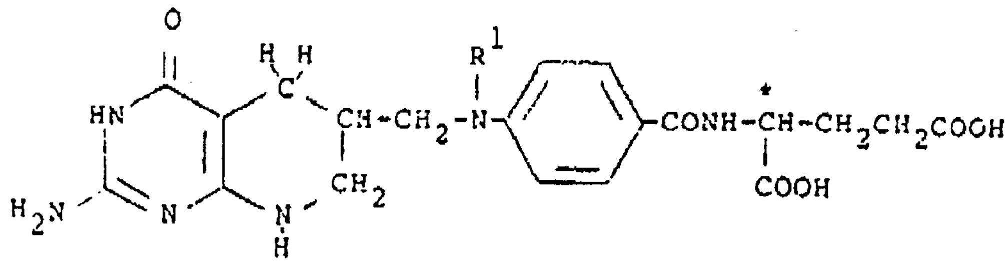

dans lequel :

$R^1$ est un hydrogène ou $R^5CO$- ;

$R^5$ est un hydrogène ou un alcoyle en $C_{1-6}$ ;

et

la configuration autour de l'atome de carbone désigné par * est L, et ses formes tautomériques, dans lesquelles on hydrolyse un composé de formule :

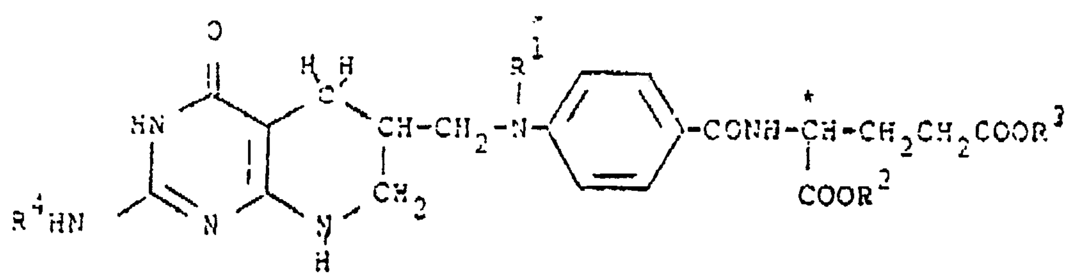

où

$R^1$ est tel que ci-dessus défini ;

$R^2$ et $R^3$ sont des groupes protecteurs d'acide carboxylique identiques ou différents ; et

$R^4$ est un groupe protecteur d'amino.

**21.** Procédé selon la revendication 20, dans lequel $R^1$ est un hydrogène, chacun des radicaux $R^2$ et $R^3$ est un alcoyle en $C_{1-6}$ et $R^4$ est un alcanoyle en $C_{2-6}$.

**22.** Procédé selon la revendication 21, dans lequel chacun des radicaux $R^2$ et $R^3$ est un éthyle et $R^4$ est un

acétyle.

**23.** Procédé selon la revendication 21 dans lequel chacun des radicaux $R^2$ et $R^3$ est un éthyle, et $R^4$ est un pivaloyle.

**24.** Procédé selon la revendication 20 dans lequel $R^1$ est un formyle, chacun des radicaux $R^2$ et $R^3$ est un alcoyle en $C_{1-6}$ et $R^4$ est un alcanol en $C_{2-6}$.

**25.** Procédé selon la revendication 24, dans lequel chacun des radicaux $R^2$ et $R^3$ est un éthyle, et $R^4$ est un acétyle.

**26.** Procédé selon la revendication 24, dans lequel chacun des radicaux $R^2$ et $R^3$ est un éthyle, et $R^4$ est un pivaloyle.

**27.** Procédé selon la revendication 20, dans lequel $R^1$ est un acétyle, chacun des radicaux $R^2$ et $R^3$ est un alcoyle en $C_{1-6}$ et $R^4$ est un alcanoyle en $C_{2-6}$.

**28.** Procédé selon la revendication 27, dans lequel chacun des radicaux $R^2$ et $R^3$ est un éthyle, et $R^4$ est un acétyle.

**29.** Procédé selon la revendication 27 dans lequel chacun des radicaux $R^2$ et $R^3$ est un éthyle et $R^4$ est un pivaloyle.

**30.** procédé de préparation d'un composé selon la revendication 1 dans lequel $R^1$ représente $R^5CO$- dans lequel on traite un composé de formule :

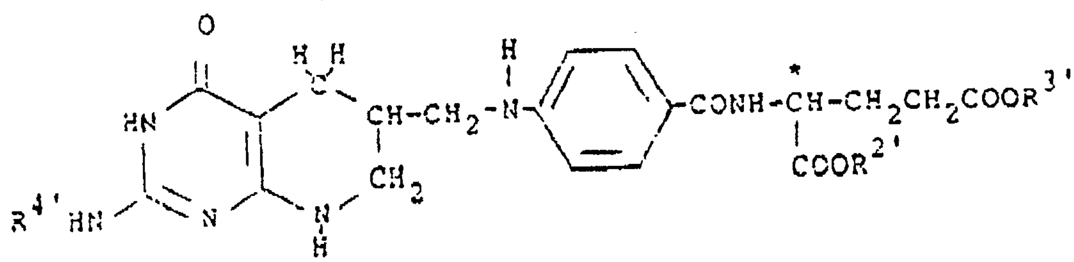

où

$R^{2'}$ et $R^{3'}$ représentent un hydrogène ou un groupe protecteur d'acide carboxylique identique ou différent ; et

$R^{4'}$ est un hydrogène ou un groupe protecteur d'amino ; et

la configuration autour de l'atome de carbone désignée par * est L,

avec un acide de formule $R^5COOH$, où $R^5$ est un hydrogène ou un alcoyle en $C_{1-6}$. ou un de ses dérivés acylants réactifs.

**31.** Procédé salon la revendication 30, dans lequel chacun des radicaux $R^{2'}$, $R^{3'}$, $R^{4'}$ et $R^5$ est un hydrogène.

**32.** Procédé selon la revendication 31, dans lequel chacun des radicaux $R^{2'}$ et $R^{3'}$ est un alcoyle en $C_{1-6'}$ $R^{4'}$ est un alcanoyle en $C_{2-6}$ et $R^5$ est un hydrogène.

**33.** Procédé de préparation d'un composé selon la revendication 1, comprenant l'hydrogénation catalytique d'un compose de formule ;

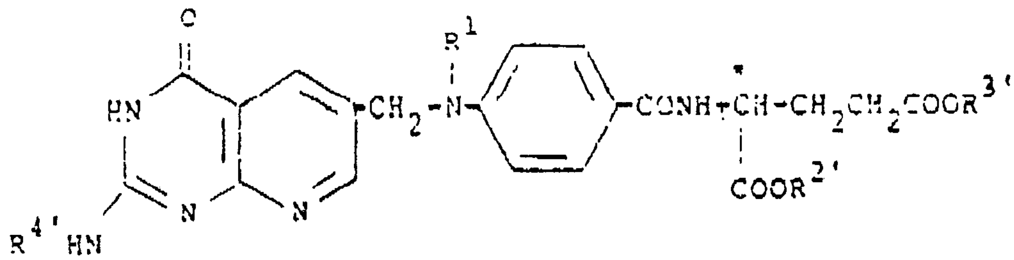

où

$R^1$, $R^{2'}$, $R^{3'}$, $R^{4'}$ sont tels qu'ici définis.

**34.** procédé selon la revendication 33, dans lequel $R^1$ est un hydrogène, chacun des radicaux $R^{2'}$ et $R^{3'}$ est un alcoyle en $C_{1-6}$ et $R^{4'}$ est un alcanoyle en $C_{2-6}$.

**35.** Procédé selon la revendication 34, dans lequel chacun des radicaux $R^{2'}$ et $R^{3'}$ est un éthyle, et $R^{4'}$ est un acétyle.

**36.** Procédé selon la revendication 34, dans lequel chacun des radicaux $R^{2'}$ et $R^{3'}$ est un éthyle, et $R^{4'}$ est un pivaloyle.

**37.** procédé selon la revendication 33, dans lequel chacun des radicaux $R^{2'}$, $R^{3'}$ et $R^{4'}$ est un hydrogène.

**38.** procédé selon la revendication 33, dans lequel $R^1$ est un formyle, chacun des radicaux $R^{2'}$ et $R^{3'}$ est un alcoyle en $C_{1-6'}$ et $R^{4'}$ est un alcanoyle en $C_{2-6}$.

**39.** Procédé selon la revendication 38, dans lequel chacun des radicaux $R^{2'}$ et $R^{3'}$ est un éthyle et $R^{4'}$ est un acétyle.

**40.** Procédé selon la revendication 38, dans lequel chacun des radicaux $R^{2'}$ et $R^{3'}$ est un éthyle et $R^{4'}$ est un pivaloyle.

**41.** Procédé selon la revendication 33, dans laquelle $R^1$ est un formyle, et chacun des radicaux $R^{2'}$ ,$R^{3'}$ et $R^{4'}$ est un hydrogène.

## Ansprüche

**1.** Eine Verbindung ausgewählt aus der Gruppe der

(i) 4(3H)-Oxo-5,6,7,8-tetrahydropyrido[2,3-d]-pyrimidine der Formel

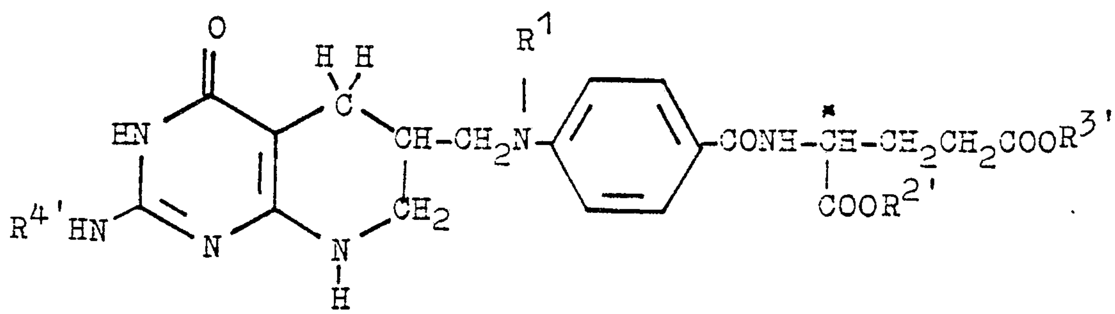

worin

$R^1$ Wasserstoff, Methyl, Ethyl oder $R^5CO$- ist,

$R^{2'}$ und $R^{3'}$ Wasserstoff oder gleiche oder verschiedene Carboxylschutzgruppen sind,

$R^{4'}$ Wasserstoff oder eine Aminoschutzgruppe,
$R^5$ Wasserstoff oder Alkyl mit 1 bis 6 Kohlenstoffatomen und
die Konfiguration an dem durch * bezeichneten Kohlenstoffatom die L-Konfiguration ist; und

(ii) tautomeren Formen dieser Pyrimidine.

**2.** Verbindung nach Anspruch 1, dadurch gekennzeichnet, daß $R^1$ Wasserstoff, $R^{2'}$ und $R^{3'}$ jeweils Wasserstoff oder Alkyl mit 1 bis 6 Kohlenstoffatomen und $R^{4'}$ Wasserstoff oder Alkanoyl mit 2 bis 6 Kohlenstoffatomen ist.

**3.** Verbindung nach Anspruch 2, dadurch gekennzeichnet, daß $R^{2'}$ und $R^{3'}$ jeweils Ethyl und $R^{4'}$ Acetyl ist.

**4.** Verbindung nach Anspruch 2, dadurch gekennzeichnet, daß $R^{2'}$ und $R^{3'}$ jeweils Ethyl und $R^{4'}$ Pivaloyl ist.

**5.** Verbindung nach Anspruch 2, dadurch gekennzeichnet, ' daß $R^{2'}$, $R^{3'}$ und $R^{4'}$ jeweils Wasserstoff ist.

**6.** Das R,S-Diastereoisomere der Verbindung nach Anspruch 5.

**7.** Das S,S-Diastereoisomere der Verbindung nach Anspruch 5.

**8.** Verbindung nach Anspruch 1, dadurch gekennzeichnet, daß $R^1$ Formyl, $R^{2'}$ und $R^{3'}$ jeweils Wasserstoff oder Alkyl mit 1 bis 6 Kohlenstoffatomen und $R^{4'}$ Wasserstoff oder Alkanoyl mit 2 bis 6 Kohlenstoffatomen ist.

**9.** Verbindung nach Anspruch 8, dadurch gekennzeichnet, daß $R^{2'}$ und $R^{3'}$ jeweils Ethyl und $R^{4'}$ Acetyl ist.

**10.** Verbindung nach Anspruch 8, dadurch gekennzeichnet, daß $R^{2'}$ und $R^{3'}$ jeweils Ethyl und $R^{4'}$ Pivaloyl ist.

**11.** Verbindung nach Anspruch 8, dadurch gekennzeichnet, daß $R^{2'}$, $R^{3'}$ und $R^{4'}$ jeweils Wasserstoff ist.

**12.** Das R,S-Diastereoisomere der Verbindung nach Anspruch 11.

**13.** Das S,S-Diastereoisomere dar Verbindung nach Anspruch 11.

**14.** Verbindung nach Anspruch 1, dadurch gekennzeichnet, daß $R^1$ Acetyl, $R^{2'}$ und $R^{3'}$ jeweils Wasserstoff oder Alkyl mit 1 bis 6 Kohlenstoffatomen und $R^{4'}$ Wasserstoff oder Alkanoyl mit 2 bis 6 Kohlenstoffatomen ist.

**15.** Verbindung nach Anspruch 14, dadurch gekennzeichnet, daß $R^{2'}$ und $R^{3'}$ jeweils Ethyl und $R^{4'}$ Acetyl ist.

**16.** Verbindung nach Anspruch 14, dadurch gekennzeichnet, daß $R^{2'}$ und $R^{3'}$ jeweils Ethyl und $R^{4'}$ Pivaloyl ist.

**17.** Verbindung nach Anspruch 14, dadurch gekennzeichnet, daß $R^{2'}$ , $R^{3'}$ und $R^{4'}$ jeweils Wasserstoff ist.

**18.** Pharmazeutische Zusammensetzung zur Bekämpfung des Wachstums von Gewebeneubildungen in einem Säuger,

gekennzeichnet durch eine Verbindung nach einem der vorstehenden Ansprüche in einer Menge, die bei der Verabreichung an den Säuger in einer Einzel- oder Mehrfachdosistherapie zur Bekämpfung des Wachstums wirksam ist, in Verbindung mit einem pharmazeutisch verträglichen Träger.

**19.** Pharmazeutische Zusammensetzung zur Bekämpfung des Wachstums von Gewebeneubildungen in einem Säuger,

gekennzeichnet durch eine Verbindung nach einem der Ansprüche 5 bis 7 oder 11 bis 13 in einer Menge, die bei der Verabreichung an den Säuger in einer Einzeloder Mehrfachdosistherapie zur Bekämpfung des Wachstums wirksam ist, in Verbindung mit einem pharmazeutisch verträglichen Träger.

**20.** Verfahren zur Herstellung einer Verbindung der Formel

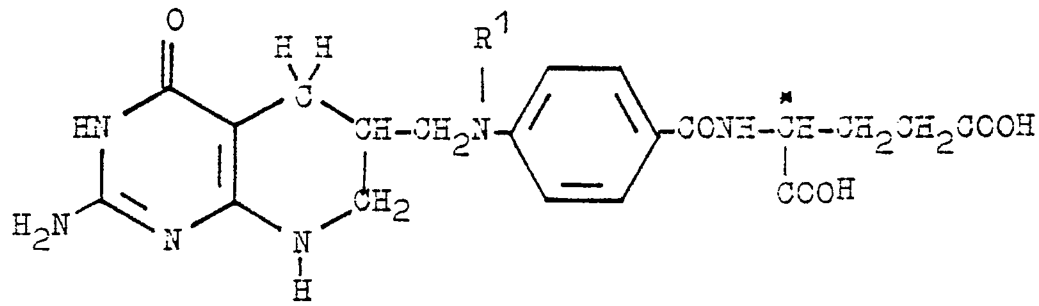

worin
$R^1$ Wasserstoff oder $R^5CO$- ,
$R^5$ Wasserstoff oder Alkyl mit 1 bis 6 Kohlenstoffatomen; und
die Konfiguration an dem durch * bezeichneten Kohlenstoffatom die L-Konfiguration ist, und deren tautomerer Formen, gekennzeichnet durch Hydrolysieren einer Verbindung der Formel

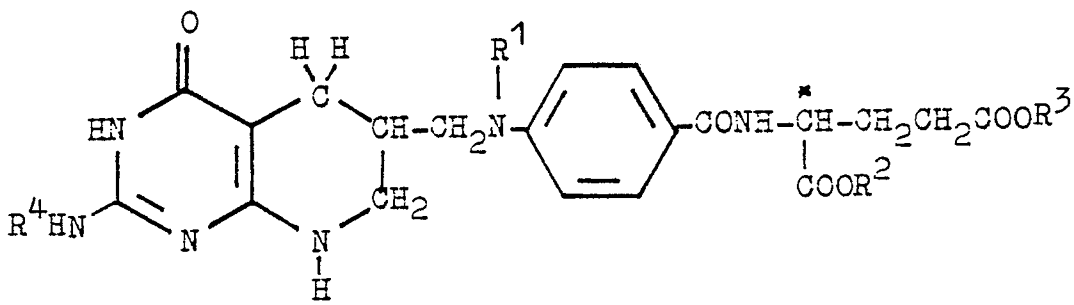

worin
$R^1$ wie hierin definiert ist,
$R^2$ und $R^3$ gleiche oder verschiedene Carboxylschutzgruppen sind und
$R^4$ eine Aminoschutzgruppe ist.

**21.** Verfahren nach Anspruch 20, dadurch gekennzeichnet, daß $R^1$ Wasserstoff, $R^2$ und $R^3$ jeweils Alkyl mit 1 bis 6 Kohlenstoffatomen und $R^4$ Alkanoyl mit 2 bis 6 Kohlenstoffatomen ist.

**22.** Verfahren nach Anspruch 21, dadurch gekennzeichnet, daß $R^2$ und $R^3$ jeweils Ethyl und $R^4$ Acetyl ist.

**23.** Verfahren nach Anspruch 21, dadurch gekennzeichnet, daß $R^2$ und $R^3$ jeweils Ethyl und $R^4$ Pivaloyl ist.

**24.** Verfahren nach Anspruch 20, dadurch gekennzeiohnet, daß $R^1$ Formyl, $R^2$ und $R^3$ jeweils Alkyl mit 1 bis 6 Kohlenstoffatomen und $R^4$ Alkanoyl mit 2 bis 6 Kohlenstoffatomen ist.

**25.** Verfahren nach Anspruch 24, dadurch gekennzeichnet, daß $R^2$ und $R^3$ jeweils Ethyl und $R^4$ Acetyl ist.

**26.** Verfahren nach Anspruch 24, dadurch gekennzeichnet, daß $R^2$ und $R^3$ jeweils Ethyl und $R^4$ Pivaloyl ist.

**27.** Verfahren nach Anspruch 20, dadurch gekennzeichnet, daß $R^1$ Acetyl, $R^2$ und $R^3$ jeweils Alkyl mit 1 bis 6 Kohlenstoffatomen und $R^4$ Alkanoyl mit 2 bis 6 Kohlenstoffatomen ist.

**28.** Verfahren nach Anspruch 27, dadurch gekennzeichnet, daß $R^2$ und $R^3$ jeweils Ethyl und $R^4$ Acetyl ist.

**29.** Verfahren nach Anspruch 27, dadurch gekennzeichnet, daß $R^2$ und $R^3$ jeweils Ethyl und $R^4$ Pivaloyl ist.

**30.** Verfahren zur Herstellung einer Verbindung nach Anspruch 1 mit $R^1$ = $R^5CO$-, dadurch gekennzeichnet, daß eine Verbindung der Formel

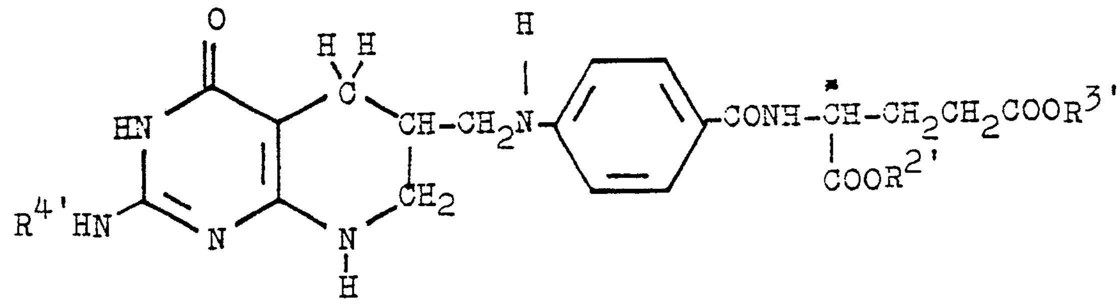

worin
$R^{2}$ und $R^{3'}$ Wasserstoff oder gleiche oder verschiedene Carboxylschutzgruppen sind,
$R^{4'}$ Wasserstoff oder eine Aminoschutzgruppe und
die Konfiguration an dem mit * bezeichneten Kohlenstoffatom die L-Konfiguration ist,

mit einer Säure der Formel $R^5COOH$, in der $R^5$ Wasserstoff oder Alkyl mit 1 bis 6 Kohlenstoffatomen ist, oder deren reaktionsfähigem Acylierungsderivat behandelt wird.

**31.** Verfahren nach Anspruch 30, dadurch gekennzeichnet, daß $R^{2'}$ , $R^{3'}$, $R^{4'}$ und $R^{5'}$ jeweils Wasserstoff ist.

**32.** Verfahren nach Anspruch 31, dadurch gekennzeichnet, daß $R^{2'}$ und $R^{3'}$ jeweils Alkyl mit 1 bis 6 Kohlenstoffatomen, $R^{4'}$ Alkanoyl mit 2 bis 6 Kohlenstoffatomen und $R^5$ Wasserstoff ist.

**33.** Verfahren zur Herstellung einer Verbindung nach Anspruch 1, gekennzeichnet durch katalytisches Hydrieren einer Verbindung der Formel

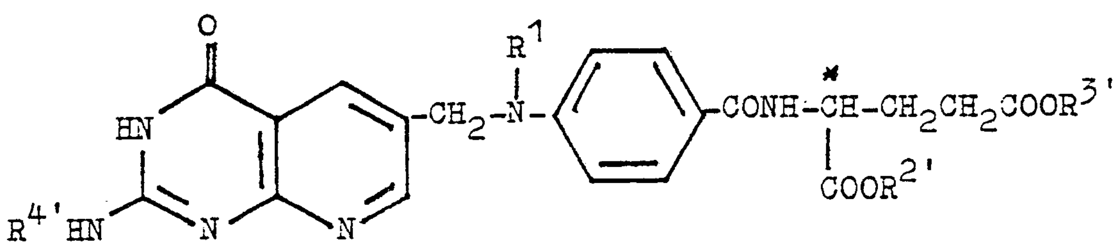

worin $R^1$, $R^{2'}$, $R^{3'}$ und $R^{4'}$ wie in Anspruch 1 definiert sind.

**34.** Verfahren nach Anspruch 33, dadurch gekennzeichnet, daß $R^1$ Wasserstoff, $R^{2'}$ und $R^{3'}$ jeweils Alkyl mit 1 bis 6 Kohlenstoffatomen und $R^{4'}$ Alkanoyl mit 2 bis 6 Kohlenstoffatomen ist.

**35.** Verfahren nach Anspruch 34, dadurch gekennzeichnet, daß $R^{2'}$ und $R^{3'}$ jeweils Ethyl und $R^{4'}$ Acetyl ist.

**36.** Verfahren nach Anspruch 34, dadurch gekennzeichnet, daß $R^{2'}$ und $R^{3'}$ jeweils Ethyl und $R^{4'}$ Pivaloyl ist.

**37.** Verfahren nach Anspruch 33, dadurch gekennzeichnet, daß $R^{2'}$ ,$R^{3'}$ und $R^{4'}$ jeweils Wasserstoff ist.

**38.** Verfahren nach Anspruch 33, dadurch gekennzeichnet, daß $R^1$ Formyl, $R^{2'}$ und $R^{3'}$ jeweils Alkyl mit 1 bis 6 Kohlenstoffatomen und $R^{4'}$ Alkanoyl mit 2 bis 6 Kohlenstoffatomen ist.

**39.** Verfahren nach Anspruch 38, dadurch gekennzeichnet, daß $R^{2'}$ und $R^{3'}$ jeweils Ethyl und $R^{4'}$ Acetyl ist.

**40.** Verfahren nach Anspruch 38, dadurch gekennzeichnet, daß $R^{2'}$ und $R^{3'}$ jeweils Ethyl und $R^{4'}$ Pivaloyl ist.

**41.** Verfahren nach Anspruch 33, dadurch gekennzeichnet, daß $R^1$ Formyl und $R^{2'}$, $R^{3'}$ und $R^{4'}$ jeweils Wasserstoff ist.